# EUROPÄISCHE PATENTSCHRIFT

(11) **EP 3 144 026 B1**
(45) Veröffentlichungstag und Bekanntmachung des Hinweises auf die Patenterteilung: **02.01.2019**
(21) Anmeldenummer: 16186841.9
(22) Anmeldetag: 01.09.2016
(51) Int. Cl.: A61M 16/06

(54) **MEHRTEILIGE BEATMUNGSMASKE, ZUGEHÖRIGES NASENSCHLUSSELEMENT UND ZUGEHÖRIGE KOMBINATION EINES SCHLAUCHANSCHLUSSELEMENTS UND EINES KOPPLUNGSELEMENTS**
MULTI-PART BREATHING MASK, ASSOCIATED NOSE CLOSURE ELEMENT AND RELATED COMBINATION OF A HOSE CONNECTING ELEMENT AND A COUPLING ELEMENT
MASQUE RESPIRATOIRE EN PLUSIEURS PARTIE, ELEMENT D'OBTURATION DU NEZ ASSOCIE ET COMBINAISON ASSOCIEE D'UN ELEMENT DE RACCORD DE TUYAU ET UN ELEMENT DE COUPLAGE

(30) Priorität: 16.09.2015 DE 102015217751
(43) Veröffentlichungstag der Anmeldung: 22.03.2017
(73) Patentinhaber: MEDIN MEDICAL INNOVATIONS GMBH, 82140 Olching (DE)
(72) Erfinder: Werner, Liselotte, 82140 Olching (DE); Weishäupl, Julia, 81547 München (DE); Schmitgen, Paul, 80802 München (DE)
(74) Vertreter: Isarpatent

(56) Entgegenhaltungen:
- DE-A1-102005 041 716
- DE-A1-102009 016 150
- US-A1- 2014 053 843

## Beschreibung

Die vorliegende Erfindung betrifft eine mehrteilige Beatmungsmaske zur Atemunterstützung eines Patienten, insbesondere eine Beatmungsmaske für Frühgeborene und Kleinkinder, gemäß dem Oberbegriff des Anspruch 1, wie aus der DE 10 2009 016 150 A1 bekannt, sowie ein zugehöriges Nasenanschlusselement und eine zugehörige Kombination eines Nasenanschlusselements und eines Kopplungselements.

Die DE 10 2010 040 769 A1 offenbart eine mehrteilige Beatmungsmaske zu Atemunterstützung eines Pateinten mit einem elastischen Nasenanschlusselement mit einem Nasenraum zum zumindest abschnittsweisen Aufnahmen der Nase des Patienten und einem starren Y-förmigen Schlauchanschlusselement mit einem ersten Zweig mit einem Gaseinlasskanal, einem zweiten Zweig mit einem Gasauslasskanal zum Anschließen einer Gasspüleinrichtung und einem dritten Zweig zum Anschließen des starren Kopplungselements, und dem starren Kopplungselement, in dem auf einer Seite der dritte Zweig des Schlauchanschlusselements drehbar verrastet ist.

Die US 2006/0283458 A1 beschreibt eine Beatmungsmaske mit einem Nasenansatzstück, einem Zwischenstück und einem Kopfbefestigungsriemen.

Die DE 10 2011 120 217 A1 beschreibt ein Nasenadaptersystem für eine CPAP-Beatmung mit einem Nasenadapter, einem Inspirationskanal und einem Exspirationskanal, einem zylindrischen Rohrelement, das verdrehbar gelagert ist, und einer Anschlusstülle.

Die US 2014/0053843 A1 beschreibt eine Beatmungsmaske mit einem Nasenanschlussstück und einem darin drehbar gelagerten Verbindungsstück zum Anschließen einer Gasquelle.

Die DE 10 2009 016 150 A1 beschreibt einen atraumatischen Nasentubus für eine nichtinvasive Atemunterstützung mit einem Zentralrohr und einem Nasenanschlussstück.

Beatmungsmasken werden unter Anderem zur Atemunterstützung in Intensivstationen verwendet. Diese Beatmungsmasken sind in unterschiedlichen Größen und Formen für die typischen Nasenformen ausgebildet, so dass im Wesentlichen für jede Nasenform eine formschlüssige Beatmungsmaske verfügbar ist. Die Beatmungsmaske weist einen Anschlussbereich zum Anschließen an eine Beatmungspumpe auf. Ein T-förmiges Adapterstück verbindet den Anschlussbereich mit zwei Schläuchen der Beatmungspumpe zum Zuführen und Abführen von Beatmungsgasen. Die Beatmungspumpe ist permanent aktiv, so dass ein fortwährender Luftstrom durch die beiden Schläuche fließt.

Der Anschlussschlauch zwischen dem Anschlussbereich der Beatmungsmaske und der Verzweigung des T-förmigen Adapterstücks wird durch die aktive Atmung eines Patienten gespült. Beim Einatmen fließt Luft von der Beatmungspumpe durch den Anschlussschlauch in die Beatmungsmaske. Beim Ausatmen presst die Lunge die verbrauchte Luft durch den Anschlussschlauch in Richtung zu der Beatmungspumpe gegen den Luftdruck der Beatmungspumpe, wo sie durch den fortwährenden Luftstrom mitgerissen wird.

Bei derartigen Beatmungsmasken atmet der Patient beim Einatmen die nach dem letzten Ausatmen in dem Anschlussschlauch verbliebene verbrauchte Luft wieder mit ein. Bei Frühgeborenen und auch bei Kleinkindern ist das Lungenvolumen gering, wie auch bei Patienten mit Lungenschäden. Der Anteil von eingeatmeter verbrauchter Luft ist bei solchen Patienten nicht vernachlässigbar. Der drohende Sauerstoffmangel wird durch Erhöhen des Sauerstoffanteils in den Beatmungsgasen kompensiert.

Die Verwendung einer derartigen Beatmungsmaske erfordert daher ein Bestimmen des Lungenvolumens des Patienten, um den Sauerstoffanteil individuell im Hinblick auf die verbrauchte Luft in dem Anschlussschlauch anzupassen. Die Anwendung derartiger Beatmungsmasken ist daher sehr aufwändig.

Ferner sind bei derartigen Beatmungsmasken der Anschlussbereich, das T-förmige Adapterstück und die Schläuche starr miteinander verbunden. Bei einer Bewegung des Patienten kann eine Zugbelastung auf die Schläuche aufgebracht werde, wodurch die Beatmungsmaske nicht mehr dicht an der Nase des Patienten anliegt. Die Funktionalität der Beatmungsmaske ist dann nicht mehr gewährleistet.

Beatmungsmasken sind beispielsweise aus der US 2006/0032500 A1, US 2005/0165334 A1 und der EP 2 428 244 A1 bekannt.

Hiervon ausgehend ist es Aufgabe der vorliegenden Erfindung, eine verbesserte mehrteilige Beatmungsmaske zur Verfügung zu stellen, welche einen stabilen Aufbau und gleichzeitig eine gute Anpassbarkeit an den Patienten sowie eine hohe Betriebssicherheit aufweist.

Diese Aufgabe wird gelöst durch eine mehrteilige Beatmungsmaske mit den Merkmalen des Patentanspruchs 1, ein zugehöriges Nasenanschlusselement mit den Merkmalen des Patentanspruchs 9 und eine zugehörige Kombination eines Schlauchanschlusselements und eines Kopplungselements mit den Merkmalen des Patentanspruchs 12.

Durch diesen Aufbau lässt sich eine mehrteilige Beatmungsmaske schaffen, welche ein weiches, elastisches Nasenanschlusselement aufweist, das sich dichtend an den Patienten anlegen lässt, wobei starre Drehgelenkelemente, realisiert durch das Kopplungselement und das Schlauchanschlusselement, eine hohe Funktionssicherheit gewährleisten. Aufgrund dieser Drehbarkeit des Schlauchanschlusselements bezüglich des Kopplungselements zueinander können der Gasauslass und der Gaseinlass einer Bewegung des Patienten folgen, wodurch Verbindungsschläuche zwischen der Beatmungsmaske und der Gasspüleinrichtung zugfrei gehalten werden können. Hierdurch ist immer ein sicherer Sitz der Beatmungsmaske auf der Nase des Patienten gewährleistet.

Das nicht-drehbare Einklemmen des Kopplungselements in das Nasenanschlusselement erhöht ebenfalls die Funktionssicherheit, da sich das Nasenanschlusselement nicht vom Kopplungselement lösen kann und zudem die Gasöffnung des Kopplungselements nicht durch eine Bewegung des Patienten verschlossen werden kann, und verringert weiterhin das Volumen an verbrauchtem Atemgas im Vorraum vor der Nase auf einen nahezu vernachlässigbaren Wert.

Vorteilhafte Ausgestaltungen und Weiterbildungen der Beatmungsmaske sind in den Unteransprüchen und den Ausführungsformen angegeben.

Gemäß einer bevorzugten Ausführungsform weist das y-förmige Schlauchanschlusselement eine innere Trennwand auf, welche den Gaseinlasskanal und den Gasauslasskanal im ersten, zweiten und dritten Zweig durchgehend trennt. Dies verringert das Volumen des Vorraums weiter.

Gemäß einer weiter bevorzugten Ausführungsform erstreckt sich der dritte Zweig des Schlauchanschlusselements im hohlzylindrischen Bereich des Kopplungselements bis zum hohlkugelförmigen Bereich. Dies verringert das Volumen des Vorraums ebenfalls weiter.

Gemäß einer weiter bevorzugten Ausführungsform weist der dritte Zweig des Schlauchanschlusselements Aussenrippen auf, die mit entsprechenden Innenrippen des hohlzylindrischen Bereichs des Kopplungselements verrastet sind. So lässt sich ein einfacher Rastmechanismus realisieren.

Gemäß einer weiter bevorzugten Ausführungsform weist der hohlkugelförmige Bereich eine zweite Auskragung auf, welche auf einer Innenwand des Aufnahmeraums abgestützt ist. Dies erhöht die Sicherheit der Verbindung zwischen Nasenanschlusselement und Kopplungselement weiter.

Gemäß einer weiter bevorzugten Ausführungsform weist das Nasenanschlusselement einen ersten Bereich mit einer Kopplungsöffnung auf, durch die das Kopplungselement in den Aufnahmeraum zum Verklemmen einführbar ist, und weist einen daran angrenzenden zweiten Bereich mit einer Nasenöffnung auf, wobei die Kopplungsöffnung und die Nasenöffnung im Wesentlichen senkrecht zueinander angeordnet sind. Diese Geometrie ist anatomisch vorteilhaft.

Gemäß einer weiter bevorzugten Ausführungsform ist ein Tragriemenanschlusselement vorgesehen, welches eine Einstecköffnung aufweist, durch die der erste Bereich des Nasenanschlusselements gesteckt ist, wobei das Tragriemenanschlusselement zwischen dem Kopplungselement und dem zweiten Bereich des Nasenanschlusselements nicht-drehbar gehaltert ist. So lässt sich das Tragriemenanschlusselement vom Nasenanschlusselement trennen und entsprechend stabiler gestalten.

Gemäß einer weiter bevorzugten Ausführungsform weist das Tragriemenanschlusselement einen ersten Tragriemenanschlussbereich und einen zweiten Tragriemenanschlussbereich auf, die über einen Verbindungsstegbereich miteinander verbunden sind, wobei der erste Tragriemenanschlussbereich die Einstecköffnung aufweist. So lässt sich eine wirksame Haltepunktanordnung mit einer Mehrzahl von über das Gesicht des Patienten verteilten Haltepunkten schaffen.

Die vorliegende Erfindung wird nachfolgend anhand bevorzugter Ausführungsformen und der beiliegenden Zeichnung erläutert. In der Zeichnung zeigen:
- Fig. 1a) - d): verschiedene Ansichten einer bevorzugten Ausführungsform einer zusammengesetzten mehrteiligen Beatmungsmaske gemäß der vorliegenden Erfindung, und zwar Fig. 1a) eine perspektivische Ansicht; Fig. 1b) eine vorderseitige Ansicht, Fig. 1c) eine rückseitige Ansicht und Fig. 1d) eine seitliche Ansicht;
- Fig. 2: eine perspektivische Ansicht der verschiedenen Elemente der mehrteiligen Beatmungsmaske nach Fig. 1a)-d) im auseinandergebauten Zustand;
- Fig. 3a), 3b): Detailansichten des Schlauchanschlusselements der mehrteiligen Beatmungsmaske nach Fig. 1a)-d), und zwar Fig. 3a) im Querschnitt und Fig. 3b) in rückseitiger Ansicht;
- Fig. 4a), b): Detailansichten des Kopplungselements der mehrteiligen Beatmungsmaske nach Fig. 1a)-d), und zwar Fig. 4a) in perspektivischer Ansicht und Fig. 4b) im Querschnitt; und
- Fig. 5a) - c): Detailansichten des Nasenanschlusselements der mehrteiligen Beatmungsmaske nach Fig. 1a)-d), und zwar Fig. 5a) in perspektivischer Ansicht, Fig. 5b) im Querschnitt und Fig. 5c) in rückseitiger Ansicht.

In den Figuren bezeichnen gleiche Bezugszeichen gleiche oder funktionsgleiche Elemente.

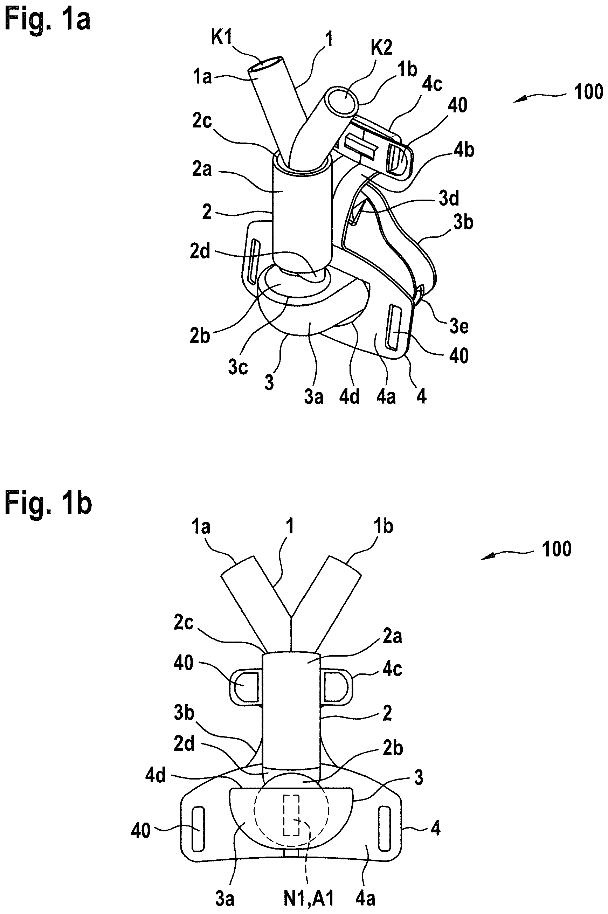

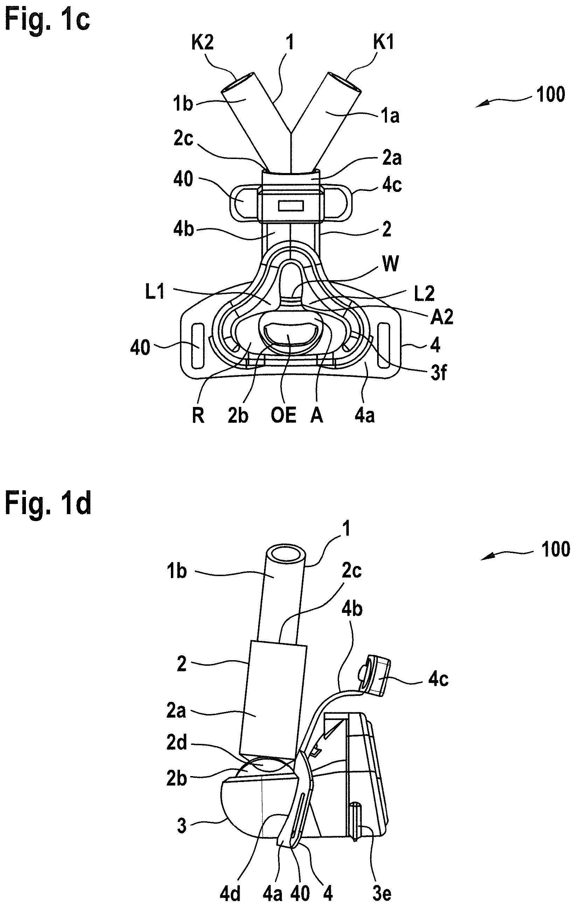

Fig. 1a) - d) zeigen verschiedene Ansichten einer bevorzugten Ausführungsform einer zusammengesetzten mehrteiligen Beatmungsmaske gemäß der vorliegenden Erfindung, und zwar Fig. 1a) eine perspektivische Ansicht; Fig. 1b) eine vorderseitige Ansicht, Fig. 1c) eine rückseitige Ansicht und Fig. 1d) eine seitliche Ansicht.

In Fig. 1a) - 1b) bezeichnet Bezugszeichen 100 eine Beatmungsmaske, welche aus einem Schlauchanschlusselement 1, einem Kopplungselement 2, einem Nasenanschlusselement 3, und einem Tragriemenanschlusselement 4 zusammengesetzt ist.

Das Schlauchanschlusselement 1, das Kopplungselement 2, und das Tragriemenanschlusselement 4 sind aus einem relativ starren Material, beispielsweise Teflon oder einem ähnlichen Kunststoff, hergestellt, um deren Stabilität zu gewährleisten. Das Nasenanschlusselement 3 ist aus einem weichen, elastischen Material hergestellt, vorzugsweise Silikon o.Ä. Die Elastizität des Nasenanschlusselements 3 ermöglicht eine gute Anpassung an das Gesicht eines (nicht dargestellten) Patienten.

Das Nasenanschlusselement 3 weist einen Nasenraum R zum zumindest abschnittsweisen Aufnehmen der Nase des Patienten und einen daran angrenzenden Aufnahmeraum A zum Aufnehmen des starren Kopplungselements 2 auf.

Das starre y-förmige Schlauchanschlusselement 1 weist einen ersten Zweig 1a mit einem Gaseinlasskanal K1, einen zweiten Zweig 1b mit einem Gasauslasskanal K2 zum Anschließen einer (nicht dargestellten) Gasspüleinrichtung sowie einen dritten Zweig 1c zum Anschließen des starren Kopplungselements 2 auf.

Das starre Kopplungselement 2 ist auf einer Seite mit dem dritten Zweig 1c des Schlauchanschlusselements 1 drehbar verrastet und auf einer anderen Seite in dem Aufnahmeraum A des Nasenanschlusselements 3 nicht-drehbar verklemmt. Die Einklemmung lässt sich durch die Gummielastizität des Nasenanschlusselements 3 über eine entsprechend dimensionierte Kopplungsöffnung 3c realisieren.

Das Tragriemenanschlusselement 4 weist eine halbkreisförmige Einstecköffnung 4d auf, durch die ein erster, schnabelförmiger Bereich 3a des Nasenanschlusselements 3 gesteckt ist, welcher ebenfalls einen halbkreisförmigen Querschnitt aufweist. Das Tragriemenanschlusselement 4 weist weiterhin einen ersten Riemenanschlussbereich 4a und einen zweiten Riemenanschlussbereich 4c auf, welche über einen Verbindungsstegbereich 4b miteinander verbunden sind.

Der erste Riemenanschlussbereich 4a weist bei diesem Beispiel die halbkreisförmige Einstecköffnung 4d auf. Bei dem derartigen Aufbau ist das Tragriemenanschlusselement 4 zwischen dem Kopplungselement 2 und einem zweiten Bereich 3b des Nasenanschlusselements 3, welcher eine Nasenöffnung 3f aufweist, nicht-drehbar gehaltert bzw. eingeklemmt. Die Kopplungsöffnung 3c und die Nasenöffnung 3f sind im Wesentlichen senkrecht zueinander angeordnet.

Bezugszeichen 3d und 3e bezeichnen optionale Verstärkungsrippen des Nasenanschlusselements 3.

Der erste Riemenanschlussbereich 4a weist beim gezeigten Beispiel zwei Löcher für Tragriemen auf, wohingegen der zweite Riemenanschlussbereich 4c drei Löcher 40 für Tragriemen aufweist. Auf diese Art und Weise lässt sich das Tragriemenanschlusselement 4 vom Nasenanschlusselement 3 entkoppeln und stabil lagern sowie mit einer entsprechenden Anordnung von Löchern 40 versehen, welche Befestigungspunkte bzw. Haltepunkte im Bereich des Gesichts des Patienten definieren.

Das Kopplungselement 2 weist einen hohlzylindrischen Bereich 2a und einen daran angrenzenden hohlkugelförmigen Bereich 2d mit einer Gasöffnung OE auf, die im zusammengebauten Zustand auf den Nasenraum R gerichtet ist. Bezugszeichen 2d bezeichnet einen (optionalen) Verstärkungsbereich zwischen dem hohlzylindrischen Bereich 2a und dem hohlkugelförmigen Bereich 2b.

Mit besonderem Bezug auf Fig. 1b) ist an der Innenwand W des Nasenanschlusselements 3 eine Nut N1 vorgesehen, in die eine erste Auskragung A1 des hohlkugelförmigen Bereichs 2b eingreift, sodass ein Verdrehen des hohlkugelförmigen Bereichs 2d im Aufnahmeraum A verhindert ist, welche unerwünschterweise einen Verschluss der Gasöffnung OE bewirken könnte.

Wie in Fig. 1c) gezeigt, weist der hohlkugelförmige Bereich 2b zusätzlich eine zweite Auskragung A2 auf, welche sich auf der Innenwand W des Nasenanschlusselements 3 abstützt, sodass ein Lösen des Kopplungselements 2 vom Nasenanschlusselement 3 noch besser verhindert werden kann.

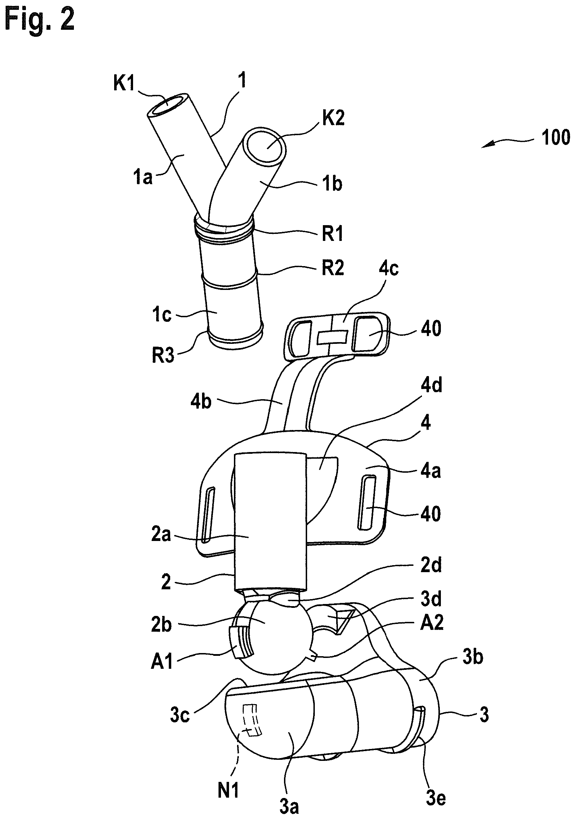

Fig. 2 zeigt eine perspektivische Ansicht der verschiedenen Elemente der mehrteiligen Beatmungsmaske nach Fig. 1a)-d) im auseinandergebauten Zustand.

Wie in Fig. 2 dargestellt, lässt sich die Beatmungsmaske 100 in die vier erwähnten Teile Schlauchanschlusselement 1, Kopplungselement 2, Nasenanschlusselement 3 und Tragriemenanschlusselement 4 zerlegen. In diesem Zusammenhang sei erwähnt, dass die Rastverbindung zwischen dem hohlzylindrischen Bereich 2a und dem dritten Zweig 1c des Schlauchanschlusselements 1 schwerer lösbar ausgelegt sein kann als die Verbindung zwischen dem hohlkugelförmigen Bereich 2b und dem Aufnahmeraum A des Nasenanschlusselements 3.

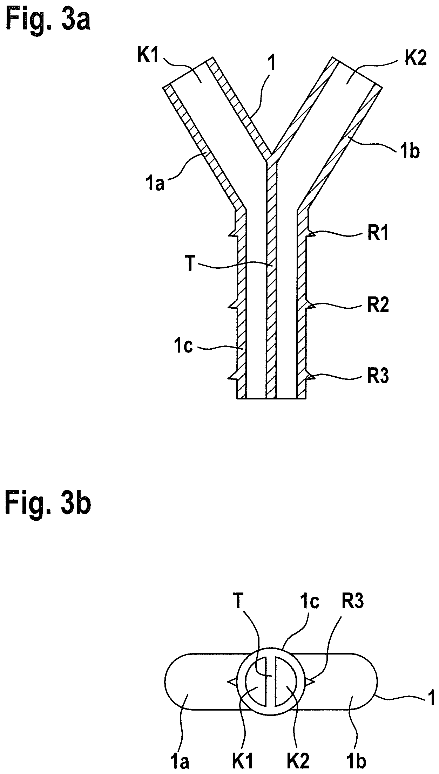

Fig. 3a), 3b) zeigen Detailansichten des Schlauchanschlusselements der mehrteiligen Beatmungsmaske nach Fig. 1a)-d), und zwar Fig. 3a) im Querschnitt und Fig. 3b) in rückseitiger Ansicht.

Wie in Fig. 3a) dargestellt, weist das Schlauchanschlusselement 1 eine innere Trennwand T auf, welche den Gaseinlasskanal K1 und den Gasauslasskanal K2 im ersten, zweiten und dritten Zweig 1a, 1b, 1c durchgehend trennt, um somit das Volumen des Vorraums möglichst gering zu halten.

Der dritte Zweig 1c des Schlauchanschlusselements 1 weist Außenrippen R1, R2, R3 in Ringform auf, die mit entsprechenden Innenrippen R4, R5, R6 des hohlzylindrischen Bereichs 2a des Kopplungselements 2 verrastet sind.

Im verrasteten Zustand erstreckt sich am gezeigten Beispiel der dritte Zweig 1c des Schlauchanschlusselements 1 im hohlzylindrischen Bereich 2a des Kopplungselements 2 bis zum hohlkugelförmigen Bereich 2b, was das Volumen des Vorraums weiter verringert.

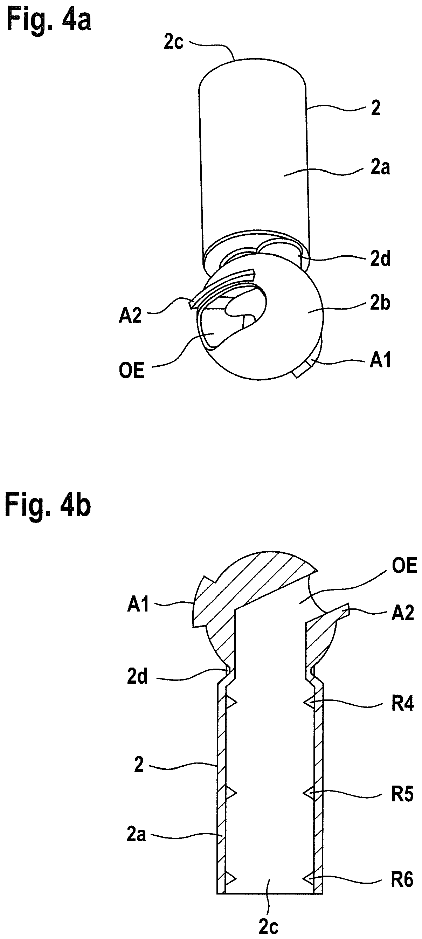

Fig. 4a), b) zeigen Detailansichten des Kopplungselements der mehrteiligen Beatmungsmaske nach Fig. 1a)-d), und zwar Fig. 4a) in perspektivischer Ansicht und Fig. 4b) im Querschnitt.

In Fig. 4a), b) ist der Aufbau des Kopplungselements 2 detailliert dargestellt, insbesondere die Anordnung der ersten Auskragung A1 und der zweiten Auskragung A2, sowie die Einstecköffnung 2c für das Schlauchanschlusselement 1 als auch der Leitungsverlauf im hohlkugelförmigen Bereich 2b, welcher im Anschluss an den eingesetzten hohlzylindrischen Bereich 2a gewinkelt durch die Gasöffnung OE in den Nasenraum R verläuft, um direkt auf die Nase des Patienten gerichtet zu sein.

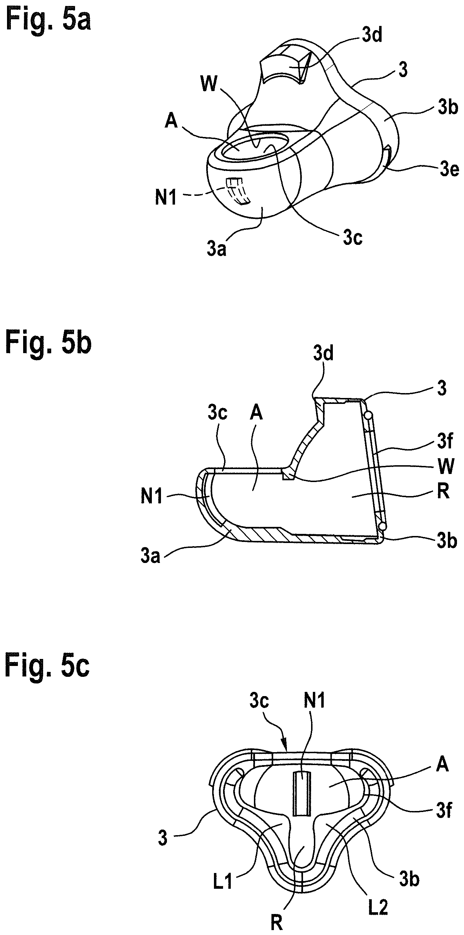

Fig. 5a) - c) zeigen Detailansichten des Nasenanschlusselements der mehrteiligen Beatmungsmaske nach Fig. 1a)-d), und zwar Fig. 5a) in perspektivischer Ansicht, Fig. 5b) im Querschnitt und Fig. 5c) in rückseitiger Ansicht.

Insbesondere sind in Fig. 5a) - c) an der Nasenöffnung 3f Dichtlippen L1, L2 erkennbar, welche die Abdichtung am Gesicht des Patienten weiter verbessern.

Obwohl die vorliegende Erfindung vorstehend in Bezug auf eine bevorzugte Ausführungsform beschreiben wurde, ist sie nicht darauf beschränkt, sondern vielfältig modifizierbar.

Insbesondere sind die dargestellten Geometrien nur beispielhaft und variierbar. Auch die Klemm-, Rast- und Gelenkverbindungen lassen sich auf viele unterschiedliche übliche Arten realisieren.

Obwohl bei dem vorstehend beschriebenen Ausführungsbeispiel ein separates Tragriemenanschlusselement vorgesehen ist, das auf den schnabelförmigen ersten Bereich des Nasenanschlusselements gesteckt ist und zwischen dem zweiten Bereich und das Kopplungselement getrennt bzw. gehaltert ist, ist dies nicht zwingend erforderlich. Je nach zu erwartender Belastung entsprechend dem Alter des Patienten können auch wie beim Stand der Technik Tragriemenanschlüsse in das Nasenanschlusselement integriert werden.

Die Anordnung der Auskragungen des halbkugelförmigen Bereichs des Kopplungselements und deren Geometrie sind ebenfalls nur beispielhaft. Insbesondere kann auch eine umgekehrte Geometrie vorgesehen werden, bei der der Aufnahmebereich Auskragungen und der halbkugelförmige Bereich des Kopplungselements Nuten aufweist bzw. eine Kombination gegenseitiger Auskragungen und Nuten.

### Bezugszeichenliste

- 100: Beatmungsmaske

- 1: Schlauchanschlusselement
- 1a: erster Zweig
- 1b: zweiter Zweig
- 1c: dritter Zweig
- K1: Gaseinlasskanal
- K2: Gasauslasskanal
- T: Trennwand
- A1: erste Auskragung
- A2: zweite Auskragung
- R1, R2, R3: Außenrippen

- 2: Kopplungselement
- 2a: hohlzylindrischer Bereich
- 2b: hohlkugelförmiger Bereich
- 2c: Einstecköffnung
- 2d: Verstärkungsbereich
- OE: Gasöffnung
- A1: erste Auskragung
- A2: zweite Auskragung
- R4, R5, R6: Innenrippen

- 3: Nasenanschlusselement
- 3a: erster Bereich
- 3b: zweiter Bereich
- 3c: Kopplungsöffnung
- 3d, 3e: Verstärkungsrippen
- 3f: Nasenöffnung
- R: Nasenraum
- A: Aufnahmeraum
- N1: Nut
- W: Innenwand
- L2, L2: Dichtlippen

- 4: Tragriemenanschlusselement
- 4a: erster Tragriemenanschlussbereich
- 4b: Verbindungsstegbereich
- 4c: zweiter Tragriemenanschlussbereich
- 4d: Einstecköffnung
- 40: Löcher für Tragriemen

## Patentansprüche

1. Mehrteilige Beatmungsmaske (100) zur Atemunterstützung eines Patienten, mit:
einem elastischen Nasenanschlusselement (3) mit einem Nasenraum (R) zum zumindest abschnittsweisen Aufnehmen der Nase des Patienten und einem daran angrenzenden Aufnahmeraum (A) zum Aufnehmen eines starren Kopplungselements (2); und
einem starren y-förmigen Schlauchanschlusselement (1) mit einem ersten Zweig (1a) mit einem Gaseinlasskanal (K1), einem zweiten Zweig (1b) mit einem Gasauslasskanal (K2) zum Anschließen einer Gasspüleinrichtung und einem dritten Zweig (1c) zum Anschließen des starren Kopplungselements (2);
**dadurch gekennzeichnet, dass**
in dem starren Kopplungselement (2), auf einer Seite der dritte Zweig (1c) des Schlauchanschlusselements (1) drehbar verrastet ist und dass das starre Kopplungselement (2) auf einer anderen Seite in dem Aufnahmeraum (A) des Nasenanschlusselements (3) nicht-drehbar verklemmt ist;
das Kopplungselement (2) einen hohlzylindrischen Bereich (2a) zum Aufnehmen des dritten Zweigs (1c) und einen daran angrenzenden hohlkugelförmigen Bereich (2b) mit einer Gasöffnung (OE) zum Einklemmen in den Aufnahmeraum (A) des Nasenanschlusselements (3) aufweist; und
der hohlkugelförmige Bereich (2b) eine erste Auskragung (A1) aufweist, welche in einer entsprechenden Nut (N1) des Aufnahmeraums (A) gelagert ist, oder dass der Aufnahmeraum (A) eine erste Auskragung aufweist, welche in einer entsprechenden Nut des hohlkugelförmigen Bereiches (2b) gelagert ist, so dass ein Verdrehen des hohlkugelförmigen Bereichs (2b) im Aufnahmeraum (A) verhindert ist.

2. Mehrteilige Beatmungsmaske (100) nach Anspruch 1, **dadurch gekennzeichnet, dass** das y-förmige Schlauchanschlusselement (1) eine innere Trennwand (T) aufweist, welche den Gaseinlasskanal (K1) und den Gasauslasskanal (K2) im ersten, zweiten und dritten Zweig (1a, 1b, 1c) durchgehend trennt.

3. Mehrteilige Beatmungsmaske (100) nach Anspruch 1, **dadurch gekennzeichnet, dass** sich der dritte Zweig (1c) des Schlauchanschlusselements (1) im hohlzylindrischen Bereich (2a) des Kopplungselements (2) bis zum hohlkugelförmigen Bereich (2b) erstreckt.

4. Mehrteilige Beatmungsmaske (100) nach einem der Ansprüche 1, 2 oder 3, **dadurch gekennzeichnet, dass** der dritte Zweig (1c) des Schlauchanschlusselements (1) Aussenrippen (R1, R2, R3) aufweist, die mit entsprechenden Innenrippen (R4, R5, R6) des hohlzylindrischen Bereichs (2a) des Kopplungselements (2) verrastet sind.

5. Mehrteilige Beatmungsmaske (100) einem der vorhergehende Ansprüche, **dadurch gekennzeichnet, dass** der hohlkugelförmige Bereich (2b) eine zweite Auskragung (A2) aufweist, welche auf einer Innenwand (W) des Aufnahmeraums (A) abgestützt ist.

6. Mehrteilige Beatmungsmaske (100) nach einem der vorhergehenden Ansprüche, **dadurch gekennzeichnet, dass** das Nasenanschlusselement (3) einen ersten Bereich (3a) mit einer Kopplungsöffnung (3c) aufweist, durch die das Kopplungselement (2) in den Aufnahmeraum (A) zum Verklemmen einführbar ist, und einen daran angrenzenden zweiten Bereich (3b) mit einer Nasenöffnung (3f) aufweist, wobei die Kopplungsöffnung (3c) und die Nasenöffnung (3f) im Wesentlichen senkrecht zueinander angeordnet sind.

7. Mehrteilige Beatmungsmaske (100) nach Anspruch 6, **dadurch gekennzeichnet, dass** ein Tragriemenanschlusselement (4) vorgesehen ist, welches eine Einstecköffnung (4d) aufweist, durch die der erste Bereich (3a) des Nasenanschlusselements (3) gesteckt ist, und dass das Tragriemenanschlusselement (4) zwischen dem Kopplungselement (2) und dem zweiten Bereich (3b) des Nasenanschlusselements (3) nicht-drehbar gehaltert ist.

8. Mehrteilige Beatmungsmaske (100) nach Anspruch 7, **dadurch gekennzeichnet, dass** das Tragriemenanschlusselement (4) einen ersten Tragriemenanschlussbereich (4a) und einen zweiten Tragriemenanschlussbereich (4c) aufweist, die über einen Verbindungsstegbereich (4b) miteinander verbunden sind, wobei der erste Tragriemenanschlussbereich (4a) die Einstecköffnung (4d) aufweist.

9. Nasenanschlusselement (3) und einem der vorherigen Ansprüche zur Verwendung für eine mehrteilige Beatmungsmaske (100) nach einem der Ansprüche 1 bis 8.

10. Nasenanschlusselement (3) nach Anspruch 9, **dadurch gekennzeichnet, dass** es einen ersten Bereich (3a) mit einer Kopplungsöffnung (3c) aufweist, durch die das Kopplungselement (2) in den Aufnahmeraum (A) zum Verklemmen einführbar ist, und einen daran angrenzenden zweiten Bereich (3b) mit einer Nasenöffnung (3f) aufweist, wobei die Kopplungsöffnung (3c) und die Nasenöffnung (3f) im Wesentlichen senkrecht zueinander angeordnet sind.

11. Nasenanschlusselement (3) nach Anspruch 9 oder 10, **dadurch gekennzeichnet, dass** es aus Silikon hergestellt ist.

12. Kombination eines Schlauchanschlusselements (1) und einem der vorherigen Ansprüche und eines Kopplungselements (2) und einem der vorherigen Ansprüche zur Verwendung für eine mehrteilige Beatmungsmaske (100) nach einem der Ansprüche 1 bis 8.

13. Kombination nach Anspruch 12, **dadurch gekennzeichnet, dass** das y-förmige Schlauchanschlusselement (1) eine innere Trennwand (T) aufweist, welche den Gaseinlasskanal (K1) und den Gasauslasskanal (K2) im ersten, zweiten und dritten Zweig (1a, 1b, 1c) durchgehend trennt.

14. Kombination nach Anspruch 12, **dadurch gekennzeichnet, dass** sich der dritte Zweig (1c) des Schlauchanschlusselements (1) im hohlzylindrischen Bereich des Kopplungselements (2) bis zum hohlkugelförmigen Bereich (2b) erstreckt.

15. Kombination nach Anspruch 12, 13 oder 14, **dadurch gekennzeichnet, dass** der dritte Zweig (1c) des Schlauchanschlusselements (1) Aussenrippen (R1, R2, R3) aufweist, die mit entsprechenden Innenrippen (R4, R5, R6) des hohlzylindrischen Bereichs (2a) des Kopplungselements (2) verrastet sind.

16. Kombination nach Anspruch 12, 13, 14 oder 15, **dadurch gekennzeichnet, dass** der hohlkugelförmige Bereich (2b) eine zweite Auskragung (A2) aufweist, welche auf einer Innenwand (W) des Aufnahmeraums (A) abgestützt ist.

## Claims

1. Multipartite respirator mask (100) for assisting a patient to breathe, with:
an elastic nose connection element (3) with a nose cavity (R) for the at least portion-wise receiving of the patient's nose and a receiving region (A) adjacent thereto for receiving a rigid coupling element (2); and
a rigid Y-shaped hose connection element (1) with a first branch (1a) with a gas inlet channel (K1), a second branch (1b) with a gas outlet channel (K2) for connecting a gas purging device and a third branch (1c) for connecting the rigid coupling element (2);
**characterised in that**
the third branch (1c) of the hose connection element (1) is rotatably engaged with one face of the rigid coupling element (2) and the rigid coupling element (2) on a different face is clamped non-rotatably in the receiving region (A) of the nose connection element (3);
the coupling element (2) exhibits a hollow cylindrical region (2a) for receiving the third branch (1c) and a hollow spherical region (2b) adjacent thereto with a gas aperture (OE) for clamping into the receiving region (A) of the nose connection element (3); and
the hollow spherical region (2b) exhibits a first projection (A1) which is mounted in a corresponding groove (N1) of the receiving region (A), or the receiving region (A) exhibits a first projection which is mounted in a corresponding groove of the hollow spherical region (2b), so preventing a twisting of the hollow spherical region (2b) in the receiving region (A).

2. Multipartite respirator mask (100) according to claim 1, **characterised in that** the Y-shaped hose connection element (1) exhibits an internal partition (T) which divides the gas inlet channel (KI) and the gas outlet channel (K2) in the first, second and third branch (1a, 1b, 1c) throughout.

3. Multipartite respirator mask (100) according to claim 1, **characterised in that** the third branch (1c) of the hose connection element (1) extends in the hollow cylindrical region (2a) of the coupling element (2) to the hollow spherical region (2b).

4. Multipartite respirator mask (100) according to any one of claims 1, 2 or 3, **characterised in that** the third branch (1c) of the hose connection element (1) exhibits external ribs (R1, R2, R3) which engage with corresponding internal ribs (R4, R5, R6) of the hollow cylindrical region (2a) of the coupling element (2).

5. Multipartite respirator mask (100) according to any one of the preceding claims, **characterised in that** the hollow spherical region (2b) exhibits a second projection (A2) which abuts against an internal wall (W) of the receiving region (A).

6. Multipartite respirator mask (100) according to any one of the preceding claims, **characterised in that** the nose connection element (3) exhibits a first region (3a) with a coupling aperture (3c) via which the coupling element (2) is insertable into the receiving region (A) for clamping, and a second region (3b) adjacent thereto with a nose aperture (3f), wherein the coupling aperture (3c) and the nose aperture (3f) are arranged substantially perpendicular to one another.

7. Multipartite respirator mask (100) according to claim 6, **characterised in that** a carrying strap connection element (4) is provided which exhibits an insertion aperture (4d) via which the first region (3a) of the nose connection element (3) is positioned, and **in that** the carrying strap connection element (4) is mounted non-rotatably between the coupling element (2) and the second region (3b) of the nose connection element (3).

8. Multipartite respirator mask (100) according to claim 7, **characterised in that** the carrying strap connection element (4) exhibits a first carrying strap connection region (4a) and a second carrying strap connection region (4c) which are interconnected by means of a connecting web region (4b), wherein the first carrying strap connection region (4a) exhibits the insertion aperture (4d).

9. Nose connection element (3) according to any one of the preceding claims for use for a multipartite respirator mask (100) according to any one of claims 1 to 8.

10. Nose connection element (3) according to claim 9, **characterised in that** it exhibits a first region (3a) with a coupling aperture (3c) via which the coupling element (2) is insertable into the receiving region (A) for clamping, and a second region (3b) adjacent thereto with a nose aperture (3f) wherein the coupling aperture (3c) and the nose aperture (3f) are arranged substantially perpendicular to one another.

11. Nose connection element (3) according to claim 9 or claim 10, **characterised in that** it is made of silicon.

12. Combination of a hose connection element (1) according to any one of the preceding claims and a coupling element (2) according to any one of the preceding claims for use for a multipartite respirator mask (100) according to any one of claims 1 to 8.

13. Combination according to claim 12, **characterised in that** the Y-shaped hose connection element (1) exhibits an internal partition (T) which divides the gas inlet channel (K1) and the gas outlet channel (K2) in the first, second and third branch (1a, 1b, 1c) throughout.

14. Combination according to claim 12, **characterised in that** the third branch (1c) of the hose connection element (1) in the hollow cylindrical region of the coupling element (2) extends to the hollow spherical region (2b).

15. Combination according to claim 12, 13 or 14, **characterised in that** the third branch (1c) of the hose connection element (1) exhibits external ribs (R1, R2, R3) which engage with corresponding internal ribs (R4, R5, R6) of the hollow cylindrical region (2a) of the coupling element (2) .

16. Combination according to claim 12, 13, 14 or 15, **characterised in that** the hollow spherical region (2b) exhibits a second projection (A2) which abuts against an internal wall (W) of the receiving region (A).

## Revendications

1. Masque respiratoire en plusieurs parties (100) pour l'assistance respiratoire d'un patient, comportant :
un élément de raccordement nasal élastique (3) avec un espace pour le nez (R) destiné à recevoir au moins par sections le nez du patient et un espace de réception (A) adjacent à celui-ci destiné à recevoir un élément d'accouplement rigide (2) ; et
un élément de raccordement de flexible (1) rigide et en forme de Y, avec une première branche (1a) comportant un canal d'admission gazeuse (K1), une deuxième branche (1b) comportant un canal de sortie gazeuse (K2) destiné à raccorder un moyen de purge gazeuse et une troisième branche (1c) destinée à raccorder l'élément d'accouplement rigide (2) ;
**caractérisé en ce que**,
dans l'élément d'accouplement rigide (2), sur un côté, la troisième branche (1c) de l'élément de raccordement de flexible (1) est encliquetée de manière rotative et **en ce que** l'élément d'accouplement rigide (2) est coincé de manière non-rotative sur un autre côté dans l'espace de réception (A) de l'élément de raccordement nasal (3) ;
l'élément d'accouplement (2) présente une zone cylindrique creuse (2a) destinée à recevoir la troisième branche (1c) et une zone sphérique creuse (2b) adjacente à celle-ci avec une ouverture gazeuse (OE) destinée à être serrée dans l'espace de réception (A) de l'élément de raccordement nasal (3) ; et
la zone sphérique creuse (2b) présente une première saillie (A1) qui est positionnée dans une rainure correspondante (N1) de l'espace de réception (A), ou **en ce que** l'espace de réception (A) présente une première saillie qui est positionnée dans une rainure correspondante de la zone sphérique creuse (2b) de manière à empêcher une rotation de la zone sphérique creuse (2b) dans l'espace de réception (A).

2. Masque respiratoire en plusieurs parties (100) selon la revendication 1, **caractérisé en ce que** l'élément de raccordement de flexible (1) en forme de y présente une cloison de séparation intérieure (T) qui sépare de manière continue le canal d'admission gazeuse (K1) et le canal de sortie gazeuse (K2) dans la première, la deuxième et la troisième branche (1a, 1b, 1c).

3. Masque respiratoire en plusieurs parties (100) selon la revendication 1, **caractérisé en ce que** la troisième branche (1c) de l'élément de raccordement de flexible (1) s'étend dans la zone cylindrique creuse (2a) de l'élément d'accouplement (2) jusqu'à la zone sphérique creuse (2b).

4. Masque respiratoire en plusieurs parties (100) selon une des revendications 1, 2 ou 3, **caractérisé en ce que** la troisième branche (1c) de l'élément de raccordement de flexible (1) présente des nervures extérieures (R1, R2, R3) qui sont encliquetées dans des nervures intérieures correspondantes (R4, R5, R6) de la zone cylindrique creuse (2a) de l'élément d'accouplement (2).

5. Masque respiratoire en plusieurs parties (100) selon une des revendications précédentes, **caractérisé en ce que** la zone sphérique creuse (2b) présente une seconde saillie (A2) qui s'appuie sur une paroi intérieure (W) de l'espace de réception (A).

6. Masque respiratoire en plusieurs parties (100) selon une des revendications précédentes, **caractérisé en ce que** l'élément de raccordement nasal (3) présente une première zone (3a) avec une ouverture d'accouplement (3c) à travers laquelle l'élément d'accouplement (2) peut être introduit dans l'espace de réception (A) pour y être coincé, et présente une seconde zone (3b) adjacente à celle-ci et présentant une ouverture nasale (3f), l'ouverture d'accouplement (3c) et l'ouverture nasale (3f) étant disposées sensiblement perpendiculairement l'une par rapport à l'autre.

7. Masque respiratoire en plusieurs parties (100) selon la revendication 6, **caractérisé en ce qu'**un élément de raccordement de sangle porteuse (4) est prévu et présente une ouverture d'insertion (4d) à travers laquelle la première zone (3a) de l'élément de raccordement nasal (3) est insérée, et **en ce que** l'élément de raccordement de sangle porteuse (4) est maintenu de manière non-rotative entre l'élément d'accouplement (2) et la seconde zone (3b) de l'élément de raccordement nasal (3).

8. Masque respiratoire en plusieurs parties (100) selon la revendication 7, **caractérisé en ce que** l'élément de raccordement de sangle porteuse (4) présente une première zone de raccordement de sangle porteuse (4a) et une seconde zone de raccordement de sangle porteuse (4c) qui sont reliées l'une à l'autre par le biais d'une zone de traverse de liaison (4b), la première zone de raccordement de sangle porteuse (4a) présentant l'ouverture d'insertion (4d).

9. Élément de raccordement nasal (3) et une des revendications précédentes pour une utilisation dans un masque respiratoire en plusieurs parties (100) selon une des revendications 1 à 8.

10. Élément de raccordement nasal (3) selon la revendication 9, **caractérisé en ce qu'**il présente une première zone (3a) comportant une ouverture d'accouplement (3c) à travers laquelle l'élément d'accouplement (2) peut être introduit dans l'espace de réception (A) pour y être coincé, et présente une seconde zone (3b) adjacente à celle-ci comportant une ouverture nasale (3f), l'ouverture d'accouplement (3c) et l'ouverture nasale (3f) étant disposées sensiblement perpendiculairement l'une par rapport à l'autre.

11. Élément de raccordement nasal (3) selon la revendication 9 ou 10, **caractérisé en ce qu'**il est réalisé en silicone.

12. Combinaison d'un élément de raccordement de flexible (1) et une des revendications précédentes et d'un élément d'accouplement (2) et une des revendications précédentes pour une utilisation dans un masque respiratoire en plusieurs parties (100) selon une des revendications 1 à 8.

13. Combinaison selon la revendication 12, **caractérisé en ce que** l'élément de raccordement de flexible (1) en forme de y présente une cloison de séparation intérieure (T) qui sépare de manière continue le canal d'admission gazeuse (K1) et le canal de sortie gazeuse (K2) dans la première, la seconde et la troisième branche (1a, 1b, 1c).

14. Combinaison selon la revendication 12, **caractérisé en ce que** la troisième branche (1c) de l'élément de raccordement de flexible (1) s'étend dans la zone cylindrique creuse de l'élément d'accouplement (2) jusqu'à la zone sphérique creuse (2b).

15. Combinaison selon la revendication 12, 13 ou 14, **caractérisé en ce que** la troisième branche (1c) de l'élément de raccordement de flexible (1) présente des nervures extérieures (R1, R2, R3) qui sont encliquetées dans des nervures intérieures correspondantes (R4, R5, R6) de la zone cylindrique creuse (2a) de l'élément d'accouplement (2).

16. Combinaison selon la revendication 12, 13, 14 ou 15, **caractérisé en ce que** la zone sphérique creuse (2b) présente une seconde saillie (A2) qui s'appuie sur une paroi intérieure (W) de l'espace de réception (A).
